**Europäisches Patentamt**

**European Patent Office**

**Office européen des brevets**

(19)

(11) Veröffentlichungsnummer : **0 204 919**
**B1**

(12) **EUROPÄISCHE PATENTSCHRIFT**

(45) Veröffentlichungstag der Patentschrift :
03.01.90

(51) Int. Cl.⁵ : **A 61 F   2/30**

(21) Anmeldenummer : **86105051.6**

(22) Anmeldetag : **12.04.86**

(54) **Femurkopfprothese.**

(30) Priorität : **12.06.85 CH 2476/85**

(43) Veröffentlichungstag der Anmeldung :
**17.12.86 Patentblatt 86/51**

(45) Bekanntmachung des Hinweises auf die Patenterteilung : **03.01.90 Patentblatt 90/01**

(84) Benannte Vertragsstaaten :
**AT DE FR GB IT**

(56) Entgegenhaltungen :
**EP–A– 0 086 879**
**EP–A– 0 115 564**
**EP–A– 0 149 527**
**WO–A–83 /025 55**
**DE–A– 2 758 541**

(73) Patentinhaber : **GEBRÜDER SULZER AKTIENGESELL-SCHAFT**
**Zürcherstrasse 9**
**CH-8401 Winterthur (CH)**

**Protek AG**
**Stadtbachstrasse 64**
**CH-3001 Bern (CH)**

(72) Erfinder : **Morscher, Erwin Walter, Prof.Dr.-med.**
**Orthopädische Universitätsklinlk Basel**
**Felix Platter Spital CH-4055 Basel (CH)**

(74) Vertreter : **Dipl.-Ing. H. Marsch Dipl.-Ing. K. Sparing**
**Dipl.-Phys.Dr. W.H. Röhl Patentanwälte**
**Rethelstrasse 123 Postfach 14 02 68**
**D-4000 Düsseldorf (DE)**

EP 0 204 919 B1

## Beschreibung

Die Erfindung betrifft eine Femurkopfprothese mit einem geraden, blattartigen Schaft, der in seinem distalen Bereich von einer Hülse radial zentriert und axial beweglich geführt ist, der weiterhin am Uebergang zwischen seinem Blatt und dem Prothesenhals einen kragenartigen Ansatz aufweist, und der schliesslich im proximalen Bereich mit Hilfe von in seine Blattseiten eingetriebenen Verankerungskeilen seitlich verkeilt ist, die lateral der Prothesenhalsachse gelegen und gegen die Schaftachse unter einem ähnlichen Winkel wie diese geneigt sind.

Aufgrund neuerer Untersuchungen — Chr.v. Hasselbach, U. Witzel « Vergleichende Spannungsanalysen an verschiedenen Hüftendoprothesen », erschienen in Zeitschrift « Unfallheilkunde » 87, 1984, Seite 205-215 — ist man bestrebt, Femurkopfprothesen im proximalen Bereich ihres Schaftes derart zu verankern, dass einerseits eine der natürlichen Belastung angenäherte Verteilung der vom Implantat auf den Knochen « fliessenden » Kräfte erreicht wird — wodurch eine Knochenatrophie besonders im Calcar-Bogen verhindert werden soll — und dass andererseits Relativbewegungen in der Grenzfläche zwischen Implantat und Knochen vermieden werden, der eigentliche Schaft jedoch in einem Gleitlager gelagert ist. Weiterhin wird gefordert, dass der hohe Elastizitätsmodul des im allgemeinen metallischen Schaftes auf dem « Weg » zu der genannten Grenzfläche an denjenigen der Knochensubstanz angenähert wird.

Aufgabe der Erfindung ist es daher, eine zementfrei zu verankernde Femurkopfprothese zu schaffen, die den vorstehenden Forderungen möglichst weitgehend genügt.

Ausgehend von der Prothese der eingangs genannten Art, wie sie beispielsweise in der EP-A-0 141 820 gezeigt und beschrieben ist, wird diese Aufgabe dadurch gelöst, dass der proximale Bereich des Schaftes medial in einer Kunststoffeinlage elastisch gelagert ist, wobei die dem Knochen zugewandte Fläche seines kragenartigen Ansatzes eine konvexe Zylinderteilfläche ist, die sich in einer konkaven Gegenfläche der Einlage abstützt, und dass ferner die Kunststoffeinlage von einem verformbaren, an der lateralen Schmalseite offenen Blechmantel umhüllt ist, dessen proximaler Rand im Bereich der Einlage zur Auflage auf der Kortikalis in der Resektionsebene des Femurs abgewinkelt ist, während der Blechmantel lateral durch die in den Blattseiten des Schaftes geführten Keile aufgeweitet und gehalten ist.

Der vorzugsweise aus Titan oder einer Titanlegierung bestehende, dünnwandige Blechmantel wird durch die Keile aufgespreizt und dadurch fest im Knochen verankert, wodurch — wie gefordert — Relativbewegungen in der Grenzfläche Knochen/Implantat vermieden sind. Diese Relativbewegungen entstehen bei der neuen Prothese einmal zwischen Blechmantel und medialer Kunststoffeinlage, in der Hauptsache jedoch zwischen dem Schaft und der Kunststoffeinlage. Diese bildet zusammen mit der distalen Hülse — die beispielsweise aus der EP-A-0 086 879 bereits im Prinzip bekannt ist — ein elastisches Lager für den Schaft, das axiale Gleitbewegungen ermöglicht. Der gegenüber dem Schaftmaterial erheblich geringere Elastizitätsmodul der Kunststoffeinlage, die vorwiegend aus Polyäthylen gefertigt ist, bewirkt gleichzeitig eine Annäherung des Elastizitätsmoduls des Gesamtimplantates an denjenigen des Knochens. Weiterhin bewirken die zylindrischen Teilflächen am kragenartigen Ansatz bzw. in der medialen Kunststoffeinlage, dass Biegemomente ein « Abrollen » des Schaftes auf der Einlage verursachen, ohne dass dabei Scherkräfte auf den Knochen ausgeübt werden. Die durch das « Abrollen » entstehenden Querkräfte infolge der auftretenden Biegemomente nimmt über die distale Hülse das kortikale Gewebe des Femurknochens auf.

Die geforderte bewegungsfreie Verankerung des Blechmantels am Knochengewebe kann gefördert werden, wenn die Aussenseite des Blechmantels mindestens teilweise mit einer das An- und Einwachsen von Gewebe fördernder Struktur versehen ist, wobei die Struktur beispielsweise in einem Drahtnetz oder -gitter bestehen kann.

Im folgenden wird die Erfindung anhand eines Ausführungsbeispiels im Zusammenhang mit der Zeichnung näher erläutert.

Fig. 1 zeigt die neue Prothese in einem in Längsrichtung geschnittenen Femurknochen, wobei die den eigentlichen Schaft im proximalen Bereich umhüllenden Elemente ebenfalls geschnitten bzw. weggelassen sind, um die Konfiguration des Schaftes sichtbar zu machen ;

Fig. 2a-Fig. 2c sind Schnitte IIa-IIa bis IIc-IIc von Fig. 1 ;

Fig. 3 ist — ohne Knochen — eine Ansicht von Fig. 1 von links ;

Fig. 4 gibt, teilweise im Schnitt und gegenüber Fig. 1 und 3 vergrössert, eine Ausführungsform für eine distale Hülse wieder ;

Fig. 5 zeigt im Schnitt V-V von Fig. 6 die mediale Kunststoffeinlage ;

Fig. 6 ist der Schnitt VI-VI von Fig. 5 ;

Fig. 7 stellt im Schnitt VII-VII von Fig. 8 den äusseren Blechmantel dar ;

Fig. 8 ist eine Aufsicht auf Fig. 7 von oben ;

Fig. 9 zeigt den Blechmantel der Fig. 7 in einer Abwicklung ;

Fig. 10a-10c bzw.

Fig. 11a-11c geben den — in Fig. 3 linken bzw. rechten — Keil zur Aufweitung des Blechmantels wieder, wobei die Darstellungen a jeweils eine Aufsicht von oben, die Darstellungen b eine Ansicht von a von unten und die Darstellungen c eine Ansicht von b von links sind.

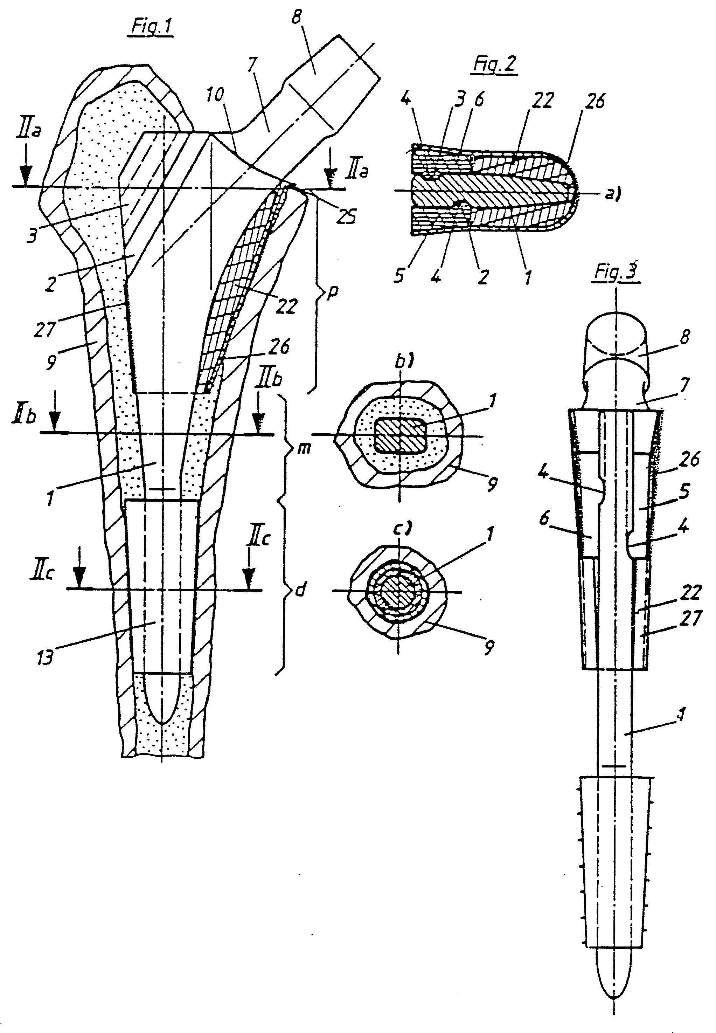

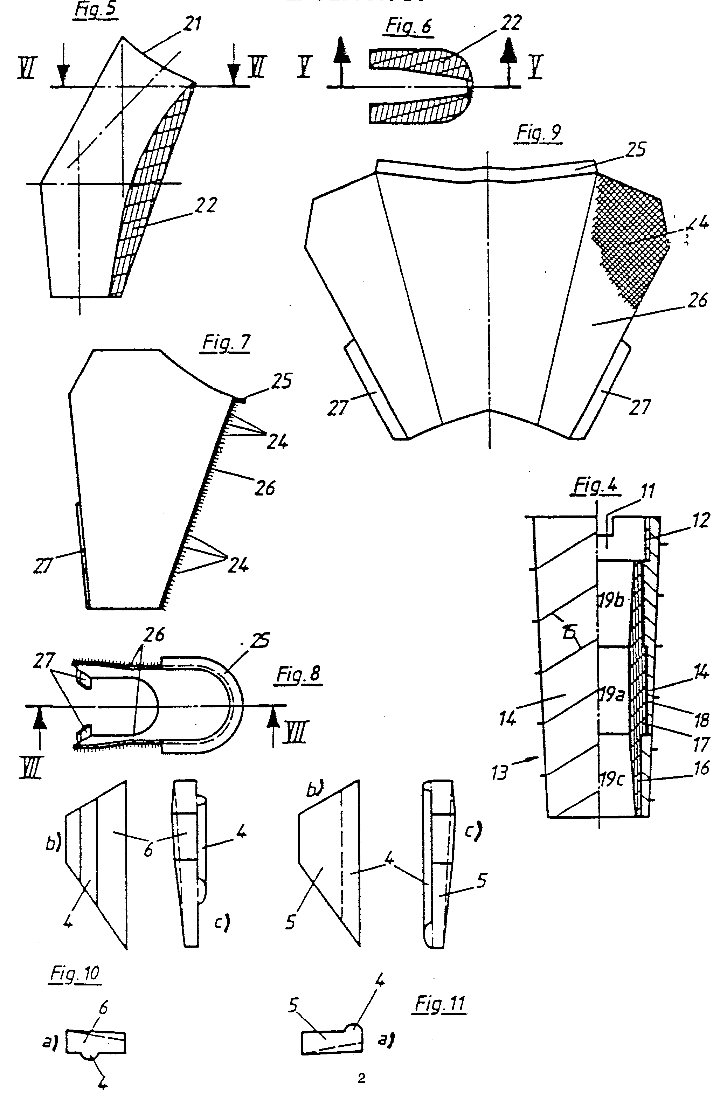

Der eigentliche, vorzugsweise aus Titan oder einer Titanlegierung gefertigte — in Fig. 1 schematisch in einen Femurknochen 9 implantiert dargestellte — Schaft 1 der Prothese hat in seinem distalen Bereich d einen konstanten kreisförmigen Querschnitt, während er über einen mittleren Uebergangsteil m, dessen Querschnitt Fig. 2b wiedergibt, in einen blattartigen Geradschaft im proximalen Bereich p übergeht.

Das Schaftblatt enthält in den nach anterior bzw. nach posterior gerichteten Blattseiten lateral je eine Nut 2 bzw. 3, in die Führungswulste 4 von Keilen 5 bzw. 6 eingreifen (Fig. 2a).

Der Schaft 1 endet proximal in einer horizontalen Schulter, die in den Prothesenhals 7 übergeht; an diesem sitzt ein konischer Zapfen 8 für die Aufnahme des nicht dargestellten Gelenkkopfes.

Zwischen den Blattseiten des Schaftes 1 und dem Prothesenhals 7 ist als Uebergang ein kragenartiger Ansatz 10 vorhanden, der in der Ansicht anterior/posterior kreiszylindrisch gerundet ist. Dieser Ansatz liegt bei der implantierten Prothese auf einer entsprechenden Schalenfläche 21 (Fig. 5) einer im wesentlichen U-förmigen Kunststoffeinlage 22 (Fig. 5) auf, die das Blatt des Schaftes 1 im Proximalen Bereich p medial umgibt.

Die mediale Kunststoffeinlage 22 ist ihrerseits aussen umhüllt von einem Blechmantel 26, der aus einem dünnen verformbaren Blech aus Reintitan oder einer Titanlegierung hergestellt ist. Dieser Blechmantel, dessen genaue Form aus den Fig. 7-9 ersichtlich ist, schliesst medial mit einem proximalen Rand 25 ab, der auf der harten Kortikalis des Knochens 9 aufliegen soll. Distal-lateral sind die Seiten des Blechmantels 26 an den Enden 27 hakenartig umgebogen. Sie umfassen so — wie ein Vergleich der Fig. 5 und 8 erkennen lässt — die Kunststoffeinlage 22. Zur Förderung des An- und Einwachsens von Knochengewebe ist der Blechmantel aussen mit einer Struktur versehen, die aus einem Drahtgitter oder Drahtnetz 24 aus Reintitan hergestellt ist.

Lateral verbleibt zwischen dem Blechmantel 26 und dem Schaft 1 im Bereich der Nuten 2 und 3 ein Zwischenraum, der mit den bereits erwähnten Keilen 5 und 6 gefüllt wird. Die Keile 5 und 6, deren Form in den Fig. 10a-10c bzw. 11a-11c dargestellt ist, werden bei der Implantation der Prothese in diesen Zwischenraum eingeschlagen. Aufgrund ihrer, aus den Fig. 10c bzw. 11c ersichtlichen, sich von distal nach proximal erweiternden Keilformen weiten die Keile 5 und 6, die mit ihrem Wulst 4 in den Nuten 2 und 3 geführt sind, den Blechmantel 26 auf und pressen ihn an die knöcherne Wand des Operationshohlraums. Auf diese Weise entsteht zwischen Mantel 26 und Knochen 9 ein inniger Kontakt, der das An- und Einwachsen von Gewebe fördert.

Die geforderte elastische Gleitlagerung wird proximal durch die Elastizität der Einlage 22 gewährleistet, auf der sich der Schaft 1 direkt ausschliesslich abstützt. Um diese Gleitlagerfähigkeit auch distal zu erhalten, ist der Schaft 1 in seinem distalen Bereich d mit seinem konstanten kreisförmigen Querschnitt in einer Führungshülse 13 gelagert. Diese weist einen metallischen Aussenmantel 14 (Fig. 4), beispielsweise aus Titan, auf; auf seiner Mantelfläche ist der Aussenmantel 14 mit einem scharfkantigen selbstschneidenden Gewinde 15 versehen, mit dem er mit Hilfe eines, einen Gewindekopf tragenden Einsetz- und Eindrehinstruments in den kortikalen Knochen eingeschraubt wird. Das Instrument, das zusätzlich in eine Nut 11 eingreift, wird dabei in ein Gewinde 12 am oberen Ende des Aussenmantels 14 eingeschraubt, ehe dieser in die Operationsöffnung im Femurknochen 9 eingesetzt wird, und ehe sein Gewinde 15 in den Knochen 9 einschneidet.

Der Aussenmantel 14 umschliesst eine Innenhülse 16 (Fig. 4) aus Polyäthylen, die in den Aussenmantel vor seiner Verankerung im Knochen 9 eingepresst ist und mit einem Vorsprung 17 in eine Vertiefung 18 des Aussenmantels 14 elastisch einschnappt. Sie dient dazu, ein direktes Reiben der beiden Metallflächen von Aussenmantel 14 und Schaft 1 zu vermeiden.

Der Hohlraum der Innenhülse 16 ist dabei dreigeteilt: Ein mittlerer zylindrischer Bereich 19a ist an den Durchmesser des distalen Schaftbereiches so angepasst, dass dieser in der Innenhülse 16 von Hand gleitend bewegt werden kann, ohne dass er in den Innenhülse 16 « taumelt ». Die äusseren Hohlraumbereiche 19b und 19c sind leicht konisch gestaltet, um — beispielsweise bei ungenauem Sitz der Hülse 13 — ein Klemmen des Schaftes 2 zu vermeiden.

## Patentansprüche

1. Femurkopfprothese mit einem geraden, blattartigen Schaft (1), der in seinem distalen Bereich (d) von einer Hülse (13) radial zentriert und axial beweglich geführt ist, der weiterhin am Uebergang zwischen seinem Blatt und dem Prothesenhals (7) einen kragenartigen Ansatz (10) aufweist, und der schliesslich im proximalen Bereich (p) mit Hilfe von in seine Blattseiten eingetriebenen Verankerungskeilen (5, 6) seitlich verkeilt ist, die lateral der Prothesenhalsachse gelegen und gegen die Schaftachse unter einem ähnlichen Winkel wie diese geneigt sind, dadurch gekennzeichnet, dass der proximale Bereich (p) des Schaftes (1) medial in einer Kunststoffeinlage (22) elastisch gelagert ist, wobei die dem Knochen (9) zugewandte Fläche seines kragenartigen Ansatzes (10) eine konvexe Zylinderteilfläche ist, die sich in einer konkaven Gegenfläche (21) der Einlage (22) abstützt, und dass ferner die Kunststoffeinlage (22) von einem verformbaren, an der lateralen Schmalseite offenen Blechmantel (26) umhüllt ist, dessen proximaler Rand (25) im Bereich der Einlage (22) zur Auflage auf der Kortikalis (9) in der Resektionsebene des Femurs abgewinkelt ist, während der Blechmantel (26) lateral durch die in den Blattseiten des Schaftes (1) geführten Keile (5, 6) aufgeweitet und gehalten ist.

2. Femurkopfprothese nach Anspruch 1, dadurch gekennzeichnet, dass die Aussenseite des Blechmantels (26) mindestens teilweise mit einer das An- und Einwachsen von Gewebe fördernder Struktur versehen ist.

3. Femurkopfprothese nach Anspruch 2, dadurch gekennzeichnet, dass die Struktur in einem Drahtnetz oder -gitter (24) besteht.

## Claims

1. A femoral head prosthesis having a straight blade-like stem (1) which in its distal zone (d) is centred radially and guided for axial movement by a sleeve (13) and which also has at the transition between its blade and the prosthesis neck (7) a collar-like projection (10) and which is wedged laterally in the proximal zone (p) by means of fixing wedges (5, 6) driven into its blade sides, the wedges 5, 6 being disposed laterally of the axis of the prosthesis neck and being inclined to the stem axis at a similar angle to the neck axis, characterised in that the proximal zone (p) of the stem (1) is resiliently mounted medially in a plastics insert (22), that surface of its collar-like projection (10) which is near the bone (9) being a convex part-cylindrical surface which bears in a matching concave surface (21) of the insert (22), and the plastics insert (22) is encased in a deformable metal casing (26) which is open on the lateral narrow side and whose proximal edge (25) is bent near the insert (22) to rest on the cortex (9) in the resection plane of the femur while the metal casing (26) is widened and retained laterally by the wedges (5, 6) guided in the blade sides of the stem (1).

2. A prosthesis according to claim 1, characterised in that the outside of the metal casing (26) has at least to some extent a structure promoting the ongrowth and invasion of tissue.

3. A prosthesis according to claim 2, characterised in that the structure takes the form of a wire network or mesh (24).

## Revendications

1. Prothèse de tête de fémur comportant une tige (1) droite, en feuille, qui dans sa région distale (d) est centrée radialement par un fourreau (13) et guidée de manière à pouvoir se déplacer axialement, et qui comporte en outre un appendice (10) en forme de collet dans la zone de transition entre sa feuille et le col (16) de la prothèse, et qui enfin est ancrée latéralement dans la région proximale (p), à l'aide de coins d'ancrage (5, 6) introduits dans les côtés de sa feuille, lesquels sont situés latéralement par rapport à l'axe du col de la prothèse et inclinés vers l'axe de la tige, sous un même angle que celui-ci, caractérisée en ce que la région proximale (p) de la tige (1) est supportée médialement de manière élastique dans un insert en matière plastique (22), la surface tournée vers l'os (9) de son appendice (10) en forme de collet étant une surface d'une partie cylindrique convexe qui prend appui dans une contre-surface (21) concave de l'insert (22), et en ce qu'en outre, l'insert en matière plastique (22) est entouré par une enveloppe de tôle (26) déformable, ouverte sur le petit côté latéral, dont le bord (25) proximal est coudé, dans la région de l'insert (22), pour reposer sur le cortex (9), dans le plan de résection du fémur, tandis que l'enveloppe de tôle (26) est élargie latéralement et maintenue par les coins (5, 6) guidés dans les côtés de la feuille de la tige (1).

2. Prothèse de tête de fémur selon la revendication 1, caractérisée en ce que la face extérieure de l'enveloppe de tôle (26) est pourvue, au moins en partie, d'une structure favorisant la croissance périphérique et interne du tissu.

3. Prothèse de tête de fémur selon la revendication 2, caractérisée en ce que la structure consiste en un filet ou en une grille métallique (24).

EP 0 204 919 B1

Fig.1

Fig.2

Fig.3

EP 0 204 919 B1

Fig. 5

Fig. 6

Fig. 9

Fig. 7

Fig. 8

Fig. 4

Fig. 10

Fig. 11

2